## Europäisches Patentamt
## European Patent Office
## Office européen des brevets

(19)

(11) Numéro de publication: **0 093 649 B1**

(12)

# FASCICULE DE BREVET EUROPEEN

(45) Date de publication du fascicule du brevet:
**28.01.87**

(21) Numéro de dépôt: **83400822.9**

(22) Date de dépôt: **26.04.83**

(51) Int. Cl.⁴: **G 21 K 1/02,** H 05 G 1/64,
A 61 B 6/02

(54) **Procédé de traitement d'image radiologique en vue de corriger ladite image des défauts dus au rayonnement diffusé.**

(30) Priorité: **04.05.82 FR 8207751**

(43) Date de publication de la demande:
**09.11.83 Bulletin 83/45**

(45) Mention de la délivrance du brevet:
**28.01.87 Bulletin 87/5**

(84) Etats contractants désignés:
**DE IT NL SE**

(56) Documents cités:
**EP - A - 0 028 036**
**DE - A - 2 452 166**
**US - A - 3 860 821**
**US - A - 3 950 613**
**US - A - 4 315 146**

(73) Titulaire: **THOMSON-CSF, 173, Boulevard Haussmann,
F-75379 Paris Cedex 08 (FR)**

(72) Inventeur: **Klausz, Rémy, THOMSON-CSF SCPI 173, bld
Haussmann, F-75379 Paris Cedex 08 (FR)**

(74) Mandataire: **Grynwald, Albert et al, THOMSON-CSF
SCPI 19, avenue de Messine, F-75008 Paris (FR)**

## Description

L'invention concerne un procédé de traitement d'une image radiologique en vue d'identifier la contribution du rayonnement X diffusé et de corriger l'image de la composante parasite qui en résulte.

Une installation de radiologie conventionnelle se compose principalement d'une source de rayons X émettant un large faisceau dans la direction d'un récepteur qui peut être un film ou un amplificateur de luminance, situé à une certaine distance de la source. La structure à examiner est placée entre la source et ce récepteur. L'image obtenue est représentative de l'absorption de rayonnement dans la structure examinée mais les contrastes sont limités par un phénomène perturbateur, à savoir la diffusion des rayons X. Il s'agit, non d'une absorption significative, mais d'une déviation de certains rayons, qui peut intervenir dans la structure à examiner tout comme dans l'amplificateur de luminance, si l'installation en comporte un. Les rayons X se propagent normalement en ligne droite sauf en cas d'interaction de diffusion en un point donné du trajet; ce point se comporte alors comme une source secondaire d'émission. En d'autres termes, le phénomène de diffusion fait que certains points du détecteur photoluminescent reçoivent des photons supplémentaires qui font défaut en d'autres ponts, ce qui nuit à la netteté de l'image.

Pour lutter contre ce phénomène, on peut disposer une grille anti-diffusante entre la structure à examiner et l'amplificateur de luminance, cette grille ayant pour effet d'absorber une partie du rayonnement diffusé dans ladite structure.

Une méthode plus performante est celle dite «des ouvertures défilantes». L'installation de radiologie est complétée en interposant deux diaphragmes à ouverture en forme de fente, lesdites fentes étant agencées et maintenues constamment homothétiques l'une de l'autre. L'un des diaphragmes est placé entre la source et la structure à examiner tandis que l'autre est placé entre ladite structure et le récepteur. Un mouvement relatif est créé entre les fentes et la structure à examiner. L'image est ainsi acquise «ligne par ligne», chaque ligne étant exempte de composante de diffusion. Un tomodensitomètre moderne peut être utilisé d'une manière qui simule la méthode des ouvertures défilantes puisque le faisceau de rayons X est plat et qu'il émet dans la direction d'une rangée de détecteurs. Il suffit alors de déplacer la structure à examiner par rapport à l'ensemble source-détecteurs sans faire tourner cet ensemble comme cela serait normalement le cas pour l'acquisition d'une image par tomodensitométrie.

A l'opposé de ces processus qui consistent en une élimination de la composante parasite de diffusion, on connaît une autre approche consistant à essayer de modéliser à priori la fonction de diffusion en tous les points de l'image et à corriger cette image en conséquence. Un article divulguant cette méthode est publié dans le N° 142 de la revue «RADIOLOGY» de Janvier 1982. Ce processus peut être efficace pour l'élimination de la composante de diffusion qui se forme dans l'amplificateur de luminance mais on peut difficilement espérer corriger de cette façon la composante parasite de diffusion qui se forme dans la structure à examiner elle-même, car la première composante est de nature optique liée à l'image alors que la seconde est une diffusion physique des rayons X dépendant de tout le volume à examiner. Or, cette seconde composante est quantitativement très importante et peut être dans certains cas du même ordre de grandeur que la composante significative d'image.

En outre, le document de brevet allemand N° 2 452 166 divulgue l'idée de mesurer en tout point la composante de diffusé au moyen d'une grille absorbante placée dans le faisceau et éliminant la moitié de ce dernier.

L'invention vise un processus tendant à modéliser la composante globale de diffusion en tous les points de l'image, c'est-à-dire une modélisation obtenue à partir d'informations prélevées dans l'image obtenue elle-même et tenant compte, par conséquent, de la composante de diffusion qui prend naissance dans la structure à examiner.

Dans cet esprit, l'invention concerne donc en premier lieu un procédé de traitement d'image radiologique d'une structure, laquelle image est obtenue à partir d'une irradiation de ladite structure par un faisceau de rayonnement pénétrant émis par une source, par exemple une source de rayonnement X, et comporte une composante significative et une composante parasite globale de diffusion, ce procédé consistant:

— à éliminer un nombre fini de parties dudit faisceau selon des directions différentes entre la source et ladite structure,

— à relever au moins une image radiologique de base dans de telles conditions;

— à déduire des régions de ladite image de base, correspondant aux parties éliminées dudit faisceau, une répartition du rayonnement diffusé sur sensiblement toute l'image, et

— à corriger ladite image de base ou éventuellement une image semblable en fonction de ladite répartition du rayonnement diffusé, et caractérisé en ce que:

— les parties éliminées dudit faisceau représentent moins de la moité de celui-ci et en ce qu'il consiste:

— à sélectionner les régions de ladite image de base correspondant aux parties éliminées dudit faisceau, à prendre en compte les intensités de rayonnement diffusé dans ces régions et à appliquer un processus d'interpolation, connu en soi, pour en déduire la répartition du rayonnement diffusé sur sensiblement toute l'image.

Par «image semblable» on entend par exemple une image complète acquise avec un faisceau complet ou une image déduite d'au moins une image de base, dans laquelle des parties manquantes ont été complétées.

De façon simple, on pourra éliminer lesdites parties dudit faisceau en interposant un masque portant un nombre fini de zones sensiblement totalement absorbantes dudit rayonnement, entre la source et la structure à examiner.

Selon un autre aspect de l'invention, la correction de ladite image de base ou de ladite image semblable sera obtenue en lui soustrayant une image représentative de ladite répartition du rayonnement diffusé.

Selon un autre aspect important de l'invention, le procédé décrit ci-dessus est complété pour obtenir une image corrigée complète. Pour cela, on peut notamment élaborer une image semblable précitée de ladite image de base en complétant cette dernière d'au moins certaines parties d'une autre image de ladite structure reproduisant au moins lesdites régions correspondant auxdites parties éliminées du faisceau, cette autre image étant prise de préférence à peu d'intervalle de temps de la première.

L'invention sera mieux comprise et d'autres avantages de celle-ci apparaîtront mieux à la lumière de la description qui va suivre d'un procédé de traitement conforme à son principe et de quelques variantes de celui-ci, donnée uniquement à titre d'exemple et faite en référence aux dessins annexés dans lesquels:

– la figure 1 illustre schématiquement une installation de radiologie incorporant les moyens nécessaires à la mise en œuvre du procédé de l'invention;

– la figure 2 illustre une installation de prise de vue et de traitement numérique des images obtenues; et

– la figure 3 illustre l'une des méthodes d'interpolation applicable dans le cadre du procédé.

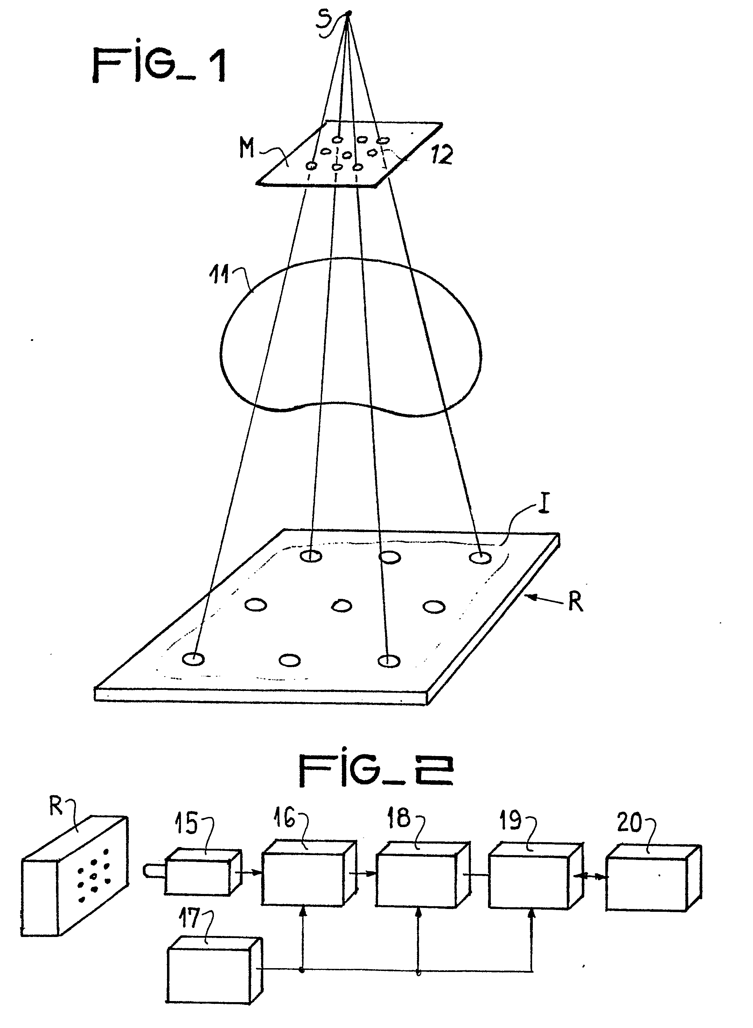

Le principe de base de l'invention se comprend aisément en examinant le schéma de la figure 1. Sur ce schéma, on a représenté une installation de radiologie comportant une source de rayonnement pénétrant S (typiquement une source de rayons X) et un récepteur R symbolisé ici par son plan de visualisation de l'image radiologique. Ce récepteur peut être en fait un amplificateur de luminance bien connu et non exempt (comme mentionné précédemment) de défauts optiques propres occasionnant une composante de diffusion supplémentaire. La structure 11 à examiner est placée entre la source S et le récepteur R.

Si on élimine un nombre fini de parties du faisceau émis par la source S, selon des directions différentes, entre ladite source et la structure 11, l'image radiologique I résultante devrait théoriquement être parsemée d'autant de régions sombres n'ayant reçu aucun rayonnement. Si au contraire on observe une certaine image, ce ne peut être que celle de la composante globale de diffusion en ces régions de l'image de base I. Partout ailleurs l'image est pratiquement identique à celle qui aurait été obtenue avec un faisceau complet puisqu'il n'y manque que la contribution du rayonnement qui aurait dû être diffusé à partir des parties éliminées du faisceau, contribution qui peut être négligée si celles-ci ne représentent qu'une faible partie du rayonnement total de la source S. Dans la pratique, on élimine un nombre fini de parties du faisceau en interposant un masque M portant un nombre fini de zones 12 considérées comme totalement absorbantes du rayonnement X, entre la source S et la structure 11, comme illustré à la figure 1. Ces zones totalement absorbantes seront constituées par des dépôts, d'épaisseur suffisante, d'un matériau de densité élevée, tel que le plomb ou le tantale. Leu épaisseur est déterminée pour provoquer une atténuation très importante dans la bande d'énergie couramment utilisée en radiologie (par exemple jusqu'à 150 keV) et en particulier une atténuation suffisante pour que le faible rayonnement résiduel transmis à travers ces dépôts soit inférieur au seuil de sensibilité du récepteur. En particulier, si, comme on le verra plus loin, l'exploitation ultérieure des images radiologiques requiert une «numérisation» de celles-ci par mots de n bits, il suffit que l'atténuation soit supérieure à la valeur que représente le bit le moins significatif. On prendra donc une atténuation égale à $k2^n$, k étant un coefficient de sécurité au moins égal à 3 ou 4. A titre d'exemple, si la numérisation s'effectue par mots de 8 bits,

$$2^n = 2^8 = 256$$

Une atténuation comprise entre $10^3$ et $10^4$ sera suffisante.

Pour une mise en œuvre simple des traitements numériques ultérieurs de l'image, on choisira un masque dont lesdites zones opaques forment une configuration géométriquement régulière, par exemple avec une périodicité suivant au moins une direction du plan du masque. Dans l'exemple de la figure 1, il s'agit simplement d'un quadrillage à maille carrée de points opaques. L'image radiologique ainsi obtenue est appelée «image de base» puisque c'est à partir de celle-ci que seront effectuées plusieurs opérations aboutissant d'une part à corriger cette image ou une image semblable pour en éliminer la composante de diffusion et d'autre part à la compléter des parties qui lui ont été nécessairement retranchées au moment de son acquisition même, par la présence du masque.

Les étapes suivantes du procédé suivant l'invention ont pour but de déduire des régions de l'image de base correspondant aux parties occultées du faisceau une répartition du rayonnement diffusé sur toute l'image. A ce stade du procédé on peut avantageusement faire appel aux techniques d'imagerie numérique consistant à effectuer les traitements voulus sur des images «numérisées» c'est-à-dire converties en informations numériques représentatives de la luminosité d'un certain nombre de points de l'image. Une installation classique de numérisation d'images radiologiques est schématisée pour mémoire à la figure 2. L'image I visible sur l'écran du récepteur R (l'amplificateur de luminance) est analysée ligne par ligne par une caméra de télévision 15 dont la sortie de signal vidéo est reliée à l'entrée d'un circuit d'échantillonnage 16 qui transforme le signal en une succession d'échelons, au rythme d'une horloge 17. Le signal échantillonné est appliqué à l'entrée d'un convertisseur analogique-numérique 18. Chaque valeur du signal vidéo échantillonné est alors transformée en un mot de n bits (n dépendant de la précision requise) et ces mots sont stockés dans une mémoire 19. La mémoire peut être relue ultérieurement et les valeurs stockées peuvent être modifiées par tout traitement approprié au moyen d'un ordinateur 20 couplé à la mémoire 19. Une visualisation finale s'effectuera en «lisant» l'image ainsi traitée et en reconstituant le signal vidéo grâce à un convertisseur numérique-analogique (non représenté) et en l'appliquant à l'entrée vidéo d'un téléviseur.

L'image de base décrite ci-dessus est donc ainsi «numérisée».

D'autre part, on acquiert une image de référence dans les mêmes conditions mais en l'absence de la structure 11 à analyser. Cette image est aussi «numérisée» et stockée de la manière suivante: on affecte la valeur 1 à tous les points où l'intensité du rayonnement reçu est inférieure à un certain seuil (ce sont les points qui correspondent aux zones absorbantes) et on affecte la valeur 0 à tous les autres points. On obtient ainsi une image du type «tout ou rien» qui va servir à effectuer une première série d'opérations sur l'image de base. L'acquisition de l'image de référence peut être faite une fois pour toutes. Il est à noter à ce stade de la description que les différentes opérations qui seront décrites ci-dessous (soustractions, moyennages point par point, additions...) ne signifient nullement que les images intermédiaires sont visualisées; on entend plutôt par «image» l'ensemble des informations numériques représentatives au départ d'une image de base ou d'une image de référence et ayant subi les mêmes transformations.

L'image de référence va servir à extraire avec précision les régions de l'image de base correspondant aux projections des zones opaques du masque. En effet, si on réalise une opération logique ET entre les parties élémentaires homologues (autrement dit point par point) des deux images stockées, le résultat est donné par la table de vérité suivante:

| Masque | Image de base | |
|---|---|---|
| 0 | $\varphi$ | 0 |
| 1 | $\varphi$ | $\varphi$ |

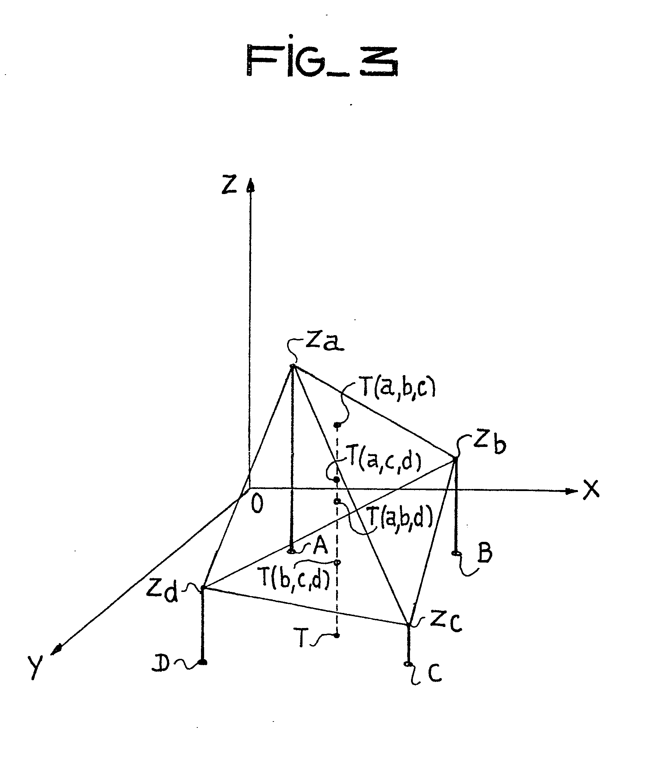

En d'autres termes, le résultat de l'opération est nul si le point correspondant du masque est transparent et il est égal à l'image de base si le point correspondant du masque est opaque aux rayons X. Cependant, les zones de l'image de base qui sont conservées par cette opération ne sont nullement représentatives d'une image de la structure 11. Elles sont au contraire l'expression de la composante globale de diffusion (intervenue entre la source et l'écran de visualisation du récepteur R) en différentes régions de l'image. A ce stade du procédé décrit, on a en fait réalisé, simultanément à l'élaboration d'une grande partie de l'image de la structure 11, un échantillonnage suffisant (au sens de la théorie de Shannon) pour reconstituer la composante de diffusion. Cette reconstitution peut s'effectuer en mettant en œuvre, par traitement numérique programmé dans l'ordinateur 20 par exemple, n'importe quel processus d'interpolation connu, la composante de diffusion en chaque point étant estimée à partir de la connaissance de celle-ci aux points d'échantillonnages les plus proches. On va décrire maintenant une méthode d'interpolation possible, en référence à la figure 3 qui représente la composante de diffusion dand un repère orthonormé tridimensionnel OXYZ. Le plan XOY représente le plan de visualisation de l'image intermédiaire résultant de l'opération ET mentionnée ci-dessus entre l'image de base et l'image de référence, étant bien entendu que cette image intermédiaire n'existe que sous forme numérique et que sa reconstruction sous forme visible ne présente pas d'intérêt. Le point T est celui où on cherche à déterminer une valeur de la composante de diffusion connaissant les valeurs de celle-ci aux points A, B, C et D les plus proches. Ces valeurs sont représentées par les hauteurs Za, Zb, Zc et Zd menées à partir des points A, B, C et D du plan OXY. La méthode d'interpolation consiste à déterminer les équations des quatre plans passant par les triplets Za, Zb, Zc–Za, Zb, Zd–Za, Zc, Zd et Zb, Zc, Zd puis les coordonnées des points d'intersection entre chacun de ces plans et la perpendiculaire au plan OXY passant par le point T. Soient: T (abc), T (abd), T (acd) et T (bcd) ces points, la valeur de la composante de diffusion au point T est déterminée en faisant la moyenne des projections de ces quatre points suivant l'axe OZ.

Toutes les opérations qui viennent d'être énoncées ci-dessus sont simples (détermination d'un plan connaissant trois points de celui-ci) et aisément programmables dans l'ordinateur 20. La capacité mémoire requise est faible dans la mesure où la valeur de la composante de diffusion en n'importe quel point situé dans le quadrilatère ABCD se déduit des équations des quatre mêmes plans, les paramètres de ces plans pouvant aisément être mis en mémoire une fois pour toutes. Bien entendu, le processus d'interpolation connu qui vient d'être rappelé ci-dessus n'est pas le seul applicable dans le cadre de l'invention. On pourra en particulier améliorer la précision en tenant compte de la connaissance du diffusé en d'autres points plus éloignés et en faisant passer par tous ces points des surfaces répondant à des équations de degré supérieur.

Lorsque ce processus d'interpolation est achevé pour tous les points d'échantillonnage de l'image intermédiaire, on obtient une image complète (sous forme numérique) de la répartition du rayonnement diffusé dans l'image radiologique. Le procédé de l'invention pourrait alors simplement être achevé en soustrayant cette image de diffusé de l'image de base mais l'image radiologique ainsi obtenue pourrait être difficilement exploitable en raison des parties de l'image de la structure 11 qui n'ont pu être acquises à cause de l'interposition du masque M. On sera donc le plus souvent amené à compléter l'image corrigée ou au contraire à appliquer globalement la correction (soustraction de l'image de répartition de diffusion) à une image de base déjà complétée.

Il est à noter que la fonction d'étalement du système impose une surface minimum à chaque zone élémentaire absorbante du masque pour que «l'ombre portée» puisse être décelée dans l'image de base. Si le masque est effectivement parsemé de zones élémentaires à surface minimum, l'image radiologique ne sera que très peu amputée et il sera suffisant de compléter les parties manquantes grâce à une interpolation faite à partir de points voisins desdites parties, dans l'image elle-même. Une autre possibilité applicable dans tous les cas consiste à ajouter au moins les parties correspondantes des parties manquantes prélevées sur une autre image, prise de préférence à peu d'intervalle de temps de la première, dont l'acquisition et la numérisation se feront après avoir enlevé ou déplacé le masque ou encore après l'avoir remplacé par un masque différent. Plusieurs solutions possibles sont décrites ci-dessous.

Selon une première variante, on peut élaborer une image complémentaire de l'image de base à partir d'un faisceau ne comportant que les parties éliminées pour l'élaboration de cette image de base, effectuer une transformation numérique de cette image complémentaire et l'additionner point par point à l'image de base corrigée. Concrètement, l'image complémentaire sera obtenue en effectuant une seconde prise de vue de la structure 11, après avoir remplacé le masque M par un masque complémentaire (non représenté) c'est-à-dire un masque totalement absorbant sauf en des zones homologues des zones absorbantes du masque M. L'acquisition de l'image complémentaire se fera à aussi peu d'intervalle de temps que possible de l'acquisition de l'image de base. Il est à noter que dans cette image très partielle de la structure 11, la composante de diffusion est très faible et peut être négligée. Il sera donc suffisant «d'ajouter» numériquement l'image complémentaire, sans lui appliquer le traitement de correction, à l'image de base corrigée. Bien entendu, le masque complémentaire doit être positionné exactement en lieu et place du masque M pour que l'image de base puisse être effectivement complétée des ses parties manquantes. Les deux prises de vue peuvent être effectuées à dose nominale.

Une autre solution peut consister à prendre purement et simplement une autre image radiologique sans masque également à aussi peu d'intervalle de temps que possible de l'image de base et à effectuer la correction sur cette image complète, l'image de base ne servant, dans ce cas, que pour déterminer la répartition de la composante de diffusion. L'image semblable complète pourra être prise à dose nominale tandis que l'image de base pourra être prise avec une dose réduite de rayonnement X, suffisante pour que la répartition de la composante de diffusion puisse être déterminée, moyennant éventuellement l'application d'un facteur correctif.

Une troisième solution consiste à relever deux images de base à partir de deux faisceaux n'ayant pas de parties éliminées communes. Concrètement, cela peut s'opérer simplement en déplaçant le masque M dans son propre plan, entre les deux prises de vue. «L'image semblable» au sens défini plus haut peut alors être obtenue en effectuant la moyenne des parties homologues des deux images de base qui ne correspondent pas aux parties éliminées de l'un ou l'autre faisceau et à «compléter» cette image intermédiaire en y ajoutant (point par point) les parties de chaque image de base qui correspondent aux parties éliminées du faisceau d'élaboration de l'autre image de base. La correction est ensuite appliquée à «l'image semblable» ainsi obtenue. Il est aussi possible d'appliquer la correction indépendamment sur chaque image de base avant de les combiner. L'intérêt de cette dernière façon de procéder réside dans le fait qu'elle offre la possibilité de déterminer deux fois «l'image» de la composante de diffusion à partir de deux positions différentes du masque et donc d'avoir un moyen de contrôle de la précision de l'algorithme d'interpolation choisi. Chaque image de base peut être prise à dose réduite.

En outre, le positionnement du masque dans l'une ou l'autre position possible peut être facilement déterminé, par exemple en acquérant deux images de référence (images du masque en l'absence de la structure 11) correspondant à ces deux positions possibles du masque. Pour des prises de vue à cadence rapide, il sera aussi possible de monter le masque sur un support mobile pour lui donner un mouvement linéaire alternatif ou un mouvement de rotation dans son propre plan et de synchroniser les prises de vue sur ce mouvement. On procédera ainsi, par exemple, à l'acquisition d'une pluralité d'images de base qui seront ensuite combinées deux par deux pour aboutir à la visualisation d'une succession d'images corrigées.

Bien entendu, l'invention n'est pas limitée au procédé décrit. D'autres modifications sont encore possibles, en particulier dans la configuration du masque. On a montré pour celui-ci une structure périodique «à pois» mais les zones absorbantes peuvent aussi avoir la forme de bandes plus ou moins larges ou de secteurs de disque (pour le cas où on déplace le masque en rotation, par exemple). Le rapport entre la surface des zones absorbantes et celle des zones non absorbantes n'est pas critique. Le procédé de l'invention est suffisamment puissant pour que la répartition de la composante de diffusion puisse encore être déterminée avec précision en utilisant un masque comportant jusqu'à 50% de zones absorbantes. Au-delà, le procédé perd de son intérêt dans la mesure où la composante de diffusion devient si faible dans la prise de vue des images de base qu'il n'est plus nécessaire de la corriger. Le problème se résume alors à la reconstruction d'une image exploitable à partir de plusieurs images de base incomplètes, technique qui rejoint, dans son principe, celle de la méthode dite «des ouvertures défilantes». C'est dire que l'invention couvre tous les équivalents techniques des moyens mis en jeu si ceux-ci le sont dans le cadre des revendications qui suivent.

## Revendications

1. Procédé de traitement d'image radiologique d'une structure (11), laquelle image (I) est obtenue à partir d'une irradiation de ladite structure par un faisceau de rayonnement pénétrant émis par une source (S) par exemple une source de rayonnement X et comporte une composante significative et une composante parasite globale de diffusion, ce procédé consistant:
— à éliminer un nombre fini de parties dudit faisceau selon des directions différentes entre la source (S) et ladite structure (11),
— à relever au moins une image radiologique de base (I) dans de telles conditions;
— à déduire des régions de ladite image de base, correspondant aux parties éliminées dudit faisceau, une répartition du rayonnement diffusé sur sensiblement toute l'image, et
— à corriger ladite image de base ou éventuellement une image semblable en fonction de ladite répartition du rayonnement diffusé, et caractérisé en ce que:
— les parties éliminées dudit faisceau représentent moins de la moitié de celui-ci et en ce qu'il consiste:
— à sélectionner les régions de ladite image de base correspondant aux parties éliminées dudit faisceau, à prendre en compte les intensités de rayonnement diffusé (Za, Zb, Zc, Zd) dans ces régions et à appliquer un processus d'interpolation, connu en soi, pour en déduire la répartition du rayonnement diffusé sur sensiblement toute l'image.

2. Procédé selon la revendication 1, caractérisé en ce qu'il consiste à éliminer lesdites parties dudit faisceau en interposant un masque (M) portant un nombre fini de zones sensiblement totalement absorbantes dudit rayonnement, entre ladite source et ladite structure.

3. Procédé selon la revendication 2, caractérisé en ce qu'on utilise un masque (M) dont lesdites zones absorbantes forment une configuration géométriquement régulière, par exemple avec une périodicité suivant au moins une direction du plan du masque.

4. Procédé selon l'une des revendications précédentes, caractérisé en ce qu'on relève une image de référence dans les mêmes conditions que ladite image de base mais en l'absence de ladite structure pour obtenir un repérage précis desdites régions de ladite image de base qui correspondent aux parties éliminées du faisceau.

5. Procédé selon l'ensemble des revendications 1 et 4, caractérisé en ce que la sélection précitée des régions de ladite image correspondant aux parties éliminées dudit faisceau consiste à réaliser une opération logique (ET) entre des parties élémentaires homologues de ladite image de base et de ladite image de référence.

6. Procédé selon l'une des revendications précédentes, caractérisé en ce qu'il consiste à corriger ladite image de base ou ladite image semblable en lui soustrayant une image représentative de ladite répartition du rayonnement diffusé.

7. Procédé selon l'une des revendications précédentes caractérisé en ce que le relevé de ladite image de base et/ou de ladite image de référence est accompagné d'une conversion sous forme numérique de celle(s)-ci et en ce qu'on effectue les opérations ultérieures précédant une visualisation de l'image corrigée précitée sur l'ensemble des données numériques ainsi obtenues.

8. Procédé selon l'une des revendications précédentes, caractérisé en ce qu'on élabore une image semblable précitée de ladite image de base en complétant cette dernière d'au moins certaines parties d'une autre image de ladite structure reproduisant au moins lesdites régions correspondant auxdites parties éliminées dudit faisceau, cette autre image étant prise de préférence à peu d'intervalle de temps de la première.

9. Procédé selon l'une des revendications 1 à 7, caractérisé en ce qu'on élabore une image complémentaire de ladite structure à partir d'un faisceau ne comportant que des parties éliminées pour l'élaboration de ladite image de base et en ce qu'on effectue une addition entre ladite image de base corrigée et ladite image complémentaire.

10. Procédé selon la revendication 9, caractérisé en ce que ladite image complémentaire est élaborée en interposant un masque complémentaire du premier masque cité, en lieu et place de celui-ci.

11. Procédé selon l'une des revendications 1 à 7, caractérisé en ce qu'il consiste à relever deux images de base à partir de deux faisceaux n'ayant aucune partie éliminée commune, à corriger chacune de ces images de base, à effectuer la moyenne des parties homologues desdites images de base corrigées qui ne correspondent pas aux parties éliminées de l'un ou l'autre faisceau et à ajouter les parties de chaque image de base corrigées qui correspondent aux parties éliminées du faisceau d'élaboration de l'autre image de base.

12. Procédé selon l'une des revendications 1 à 8, caractérisé en ce qu'il consiste à relever deux images de base à partir de deux faisceaux n'ayant aucune partie éliminée commune, à effectuer la moyenne des parties homologues desdites images de base qui ne correspondent pas aux parties éliminées de l'un ou l'autre faisceau, à y ajouter les parties de chaque image de base qui correspondent aux parties éliminées du faisceau d'élaboration de l'autre image de base de façon à obtenir ladite image semblable et à corriger cette dernière.

13. Procédé selon l'une des revendications 11 ou 12, caractérisé en ce que les deux images de base sont prises à peu d'intervalle de temps l'une de l'autre en interposant un masque portant un nombre fini de zones sensiblement totalement absorbantes dudit rayonnement entre ladite source et l'objet et en déplaçant ce masque entre les deux prises de vue desdites images de base.

14. Procédé selon la revendications 13, caractérisé en ce que chacune des deux images de base est prise avec une dose de rayonnement réduite.

15. Procédé selon la revendication 13 ou 14, caractérisé en ce que ledit masque est déplacé dans son propre plan suivant un mouvement linéaire alternatif ou un mouvement rotatif et en ce que les prises de vue des deux images de base sont synchronisées sur ce mouvement.

16. Procédé selon l'une des revendications 1 à 7, caractérisé en ce qu'on élabore une image semblable précitée par une seconde prise de vue de ladite structure faite à peu d'intervalle de temps de la prise de vue de ladite image de base, cette seconde prise de vue étant effectuée à partir d'un faisceau d'irradiation complet et en ce qu'on corrige l'image semblable complète qui en résulte.

17. Procédé selon l'une des revendications 1 à 7 caractérisé en ce qu'on complète les parties manquantes d'une image de base éventuellement corrigée au moyen d'une interpolation, connue en soi, faite à partir de points voisins desdites parties manquantes, dans l'image elle-même.

**Claims**

1. Processing method for a radiologic image of a structure (11), wherein the image (I) is obtained from an irradiation of said structure by a beam of penetrating radiation emitted by a source (S), for example an X-ray source, and comprises a significant component and a parasitic total diffusion component, this method consisting in:
— eliminating a finite number of portions of said beam in different directions between the source (S) and said structure (11),
— taking at least one radiologic base image (I) in these conditions;
— deriving from the regions of said base image corresponding to the eliminated portions of said beam a distribution of the diffused radiation across substantially the entire image; and
— correcting said base image or eventually a similar image in function of said distribution of the diffused radiation, and characterized in that:
— the eliminated portions of said beam represent less than half of the latter, and in that it consists in:
— selecting those regions of said base image corresponding to the eliminated portions of said beam, taking into account the diffused radiation intensities ($Z_a$, $Z_b$, $Z_c$, $Z_d$) in those regions, and applying an interpolation process, known per se, to derive therefrom the distribution of diffused radiation substantially across the entire image.

2. Method according to claim 1, characterized in that it consists in eliminating said portions of said beam by interposing a mask (M) carrying a finite number of zones which substantially totally absorb said radiation between said source and said structure.

3. Method according to claim 2, characterized in that a mask (M) is used the absorbing zones of which form a regular geometric configuration, for example with a periodicity in at least one direction of the plane of the mask.

4. Method according to any of the preceding claims, characterized in that a reference image is taken in the same conditions as said base image, but in the absence of said structure, to obtain a precise localization of said regions of said base image which correspond to the eliminated portions of said beam.

5. Method according to the combined claims 1 and 4, characterized in that the above-mentioned selection of the regions of said image corresponding to the eliminated portions of said beams consists in performing a logic operation (AND) between the mutually corresponding elementary portions of said base image and said reference image.

6. Method according to any of the preceding claims, characterized in that it consists in correcting said base image or said similar image by subtracting therefrom an image which is representative of said distribution of the diffused radiation.

7. Method according to any of the preceding claims, characterized in that the taking of said base image and/or said reference image is accompanied by a digitalization thereof and in that the subsequent operations preceding a visualization of the corrected above-mentioned image are carried out on the totality of the thus obtained digital data.

8. Method according to any of the preceding claims, characterized in that an above-mentioned image similar to said base image is generated by completing the latter with at least certain portions of another image of said structure reproducing at least said regions corresponding to said eliminated portions of said beam, this other image being taken, preferentially, at a short time interval from the first.

9. Method according to any of claims 1 to 7, characterized in that an image which is complementary to said structure is generated from a beam containing only the portions eliminated for the generation of said base image, and in that said corrected base image is added to said complementary image.

10. Method according to claim 9, characterized in that said complementary image is generated by interposing a mask which is complementary of the first-mentioned mask instead of the latter.

11. Method according to any of claims 1 to 7, characterized in that it consists in taking two base images from two beams not having any common eliminated portion, correcting each of these base images, forming the average of the mutually corresponding portions of said correct base images which do not correspond to the eliminated portions of one or the other beam, and adding the portions of each corrected base image which correspond to the eliminated portions of the beam for the generation of the other base image.

12. Method according to any of claims 1 to 8, characterized in that it consists in taking two base images from two beams which do not have any common eliminated portion, forming the average of the mutually corresponding portions of said base images which do not correspond to the eliminated portions of one or the other beam, adding those portions of each base image which correspond to the eliminated portions of the beam for the generation of the other base image, in a manner to obtain said similar image, and correcting the latter.

13. Method according to any of claims 11 and 12, characterized in that the two base images are taken at a short time interval from each other, a mask carrying a finite number of zones which substantially totally absorb said radiation being interposed between said source and the object, and said mask being moved between the two takings of said base images.

14. Method according to claim 13, characterized in that each of the two base images is taken at reduced radiation dosage.

15. Method according to claims 13 or 14, characterized in that said mask is moved in its own plane with an alternating linear movement or with a rotary movement, and in that the takings of the two base images are synchronized with this movement.

16. Method according to any of claims 1 to 7, characterized in that an above-mentioned similar image is generated by a second recording of said structure taken at a short time interval from the taking of said base image, this second recording being performed with a complete irradiation beam, and in that the complete similar image resulting therefrom is corrected.

17. Method according to any of claims 1 to 7, characterized in that the missing portions of a base image are completed, the latter being eventually corrected by means of an interpolation which is known per se and obtained from adjacent points of said missing portions within the image itself.

**Patentansprüche**

1. Verfahren zur Aufbereitung eines radiologischen Bildes einer Struktur (11), bei welchem das Bild (I) dadurch erhalten wird, dass diese Struktur durch ein Bündel durchdringender Strahlung bestrahlt wird, das von einer Quelle (S) ausgesendet wird, beispielsweise einer Röntgenquelle, und eine signifikante Komponente sowie eine globale Streuungs-Störkomponente enthält, wobei dieses Verfahren darin besteht, dass:
– eine endliche Anzahl von Teilen des Bündels in verschiedenen Richtungen zwischen der Quelle (S) und der Struktur (11) unterdrückt wird,
– unter diesen Bedingungen wenigstens ein radiologisches Grundbild (I) aufgenommen wird;
– aus denjenigen Bereichen des genannten Grundbildes, welche den unterdrückten Teilen des Bündels entsprechen, eine Verteilung der Streustrahlung über im wesentlichen das gesamte Bild abgeleitet wird; und

– das genannte Grundbild oder gegebenenfalls ein ähnliches Bild in Abhängigkeit von der genannten Verteilung der Streustrahlung korrigiert wird, dadurch gekennzeichnet, dass
– die unterdrückten Teile des Bündels weniger als die Hälfte desselben darstellen und dass es darin besteht,
– diejenigen Bereiche des genannten Grundbildes auszuwählen, welche den unterdrückten Teilen des Bündels entsprechen, die Streustrahlungsintensitäten ($Z_a$, $Z_b$, $Z_c$, $Z_d$) in diesen Bereichen zu berücksichtigen und einen an sich bekannten Interpolationsprozess anzuwenden, um daraus die Verteilung der Streustrahlung über im wesentlichen das gesamte Bild abzuleiten.

2. Verfahren nach Anspruch 1, dadurch gekennzeichnet, dass es darin besteht, die genannten Teile des Bündels dadurch zu unterdrücken, dass eine Maske (M) zwischen der Quelle und der genannten Struktur eingefügt wird, welche eine endliche Anzahl von für die genannte Strahlung praktisch vollständig absorbierenden Zonen enthält.

3. Verfahren nach Anspruch 2, dadurch gekennzeichnet, dass eine Maske (M) verwendet wird, deren absorbierende Zonen eine gleichmässige geometrische Konfiguration bilden, beispielsweise mit einer Periodizität in wenigstens einer Richtung der Ebene der Maske.

4. Verfahren nach einem der vorstehenden Ansprüche, dadurch gekennzeichnet, dass ein Referenzbild unter denselben Bedingungen wie das Grundbild aufgenommen wird, jedoch in Abwesenheit der genannten Struktur, um eine genaue Markierung der genannten Bereiche des genannten Grundbildes, welche den unterdrückten Teilen des Bündels entsprechen, zu erhalten.

5. Verfahren nach der Gesamtheit der Ansprüche 1 und 4, dadurch gekennzeichnet, dass die vorgenannte Auswahl der Bereiche des genannten Bildes, welche den unterdrückten Teilen des Bündels entsprechen, darin besteht, eine Logikoperation (UND) durchzuführen zwischen den einander entsprechenden Elementarteilen des genannten Grundbildes und des genannten Referenzbildes.

6. Verfahren nach einem der vorstehenden Ansprüche, dadurch gekennzeichnet, dass es darin besteht, das genannte Grundbild oder das genannte ähnliche Bild zu korrigieren, indem von ihm ein Bild subtrahiert wird, welches repräsentativ für die genannte Verteilung der Streustrahlung ist.

7. Verfahren nach einem der vorstehenden Ansprüche, dadurch gekennzeichnet, dass die Aufnahme des genannten Grundbildes und/oder des genannten Referenzbildes unter Umsetzung desselben bzw. derselben in Digitalform geschieht und dass die weiteren Operationen, welche der Sichtanzeige des genannten korrigierten Bildes vorausgehen, auf die Gesamtheit von so erhaltenen Digitaldaten angewendet werden.

8. Verfahren nach einem der vorstehenden Ansprüche, dadurch gekennzeichnet, dass ein vorerwähntes Bild, welches dem Grundbild ähnlich ist, erzeugt wird, indem letzteres vervollständigt wird durch wenigstens bestimmte Teile eines anderen Bildes der genannten Struktur, welche wenigstens diejenigen Bereiche wiedergeben, die den unterdrückten Teilen des Bündels entsprechen, wobei dieses andere Bild vorzugsweise in geringem Zeitabstand von dem ersteren aufgenommen wird.

9. Verfahren nach einem der Ansprüche 1 bis 7, dadurch gekennzeichnet, dass ein zu der genannten Struktur komplementäres Bild erzeugt wird durch ein Bündel, welches nur die für die Erzeugung des genannten Grundbildes unterdrückten Teile enthält, und dass eine Addition des genannten korrigierten Grundbildes zu dem genannten komplementären Bild durchgeführt wird.

10. Verfahren nach Anspruch 9, dadurch gekennzeichnet, dass das genannte komplementäre Bild erzeugt wird, indem eine Maske, die komplementär zu der erstgenannten Maske ist, anstelle derselben eingesetzt wird.

11. Verfahren nach einem der Ansprüche 1 bis 7, dadurch gekennzeichnet, dass es darin besteht, zwei Grundbilder mittels zwei Bündeln aufzunehmen, die keinerlei gemeinsamen unterdrückten Teil aufweisen, jedes dieser Grundbilder zu korrigieren, den Mittelwert der einander entsprechenden Teile der korrigierten Grundbilder zu bilden, welche nicht den unterdrückten Teilen des einen oder anderen Bündels entsprechen, und die korrigierten Teile jedes Grundbildes hinzuzufügen, welche den unterdrückten Teilen des Bündels für die Erzeugung des anderen Grundbildes entsprechen.

12. Verfahren nach einem der Ansprüche 1 bis 8, dadurch gekennzeichnet, dass es darin besteht, zwei Grundbilder mittels zwei Bündeln aufzunehmen, die keinerlei gemeinsamen unterdrückten Teil aufweisen, den Mittelwert der einander entsprechenden Teile der genannten Grundbilder zu bilden, welche nicht den unterdrückten Teilen des einen oder anderen Bündels entsprechen, die Teile jedes Grundbildes hinzuzufügen, welche den unterdrückten Teilen des Bündels für die Erzeugung des anderen Grundbildes entsprechen, so dass das genannte ähnliche Bild erhalten wird, und letzteres zu korrigieren.

13. Verfahren nach Anspruch 11 oder 12, dadurch gekennzeichnet, dass die zwei Grundbilder in geringem Zeitabstand voneinander aufgenommen werden, indem eine Maske zwischen der Quelle und dem Objekt eingefügt wird, welche eine endliche Anzahl von für die Strahlung praktisch vollständig absorbierenden Zonen trägt, wobei diese Maske zwischen den beiden Aufnahmen der Grundbilder bewegt wird.

14. Verfahren nach Anspruch 13, dadurch gekennzeichnet, dass jedes der zwei Grundbilder mit einer verminderten Strahlungsdosis aufgenommen wird.

15. Verfahren nach Anspruch 13 oder 14, dadurch gekennzeichnet, dass die genannte Maske in ihrer eigenen Ebene mit einer wechselnden Linearbewegung oder einer Drehbewegung verschoben wird und dass die Aufnahmen der zwei Grundbilder mit dieser Bewegung synchronisiert werden.

16. Verfahren nach einem der Ansprüche 1 bis 7, dadurch gekennzeichnet, dass ein vorerwähntes ähnliches Bild erzeugt wird durch eine zweite Aufnahem der genannten Struktur in geringem Zeitabstand von der Aufnahme des genannten Grundbildes, wobei diese zweite Aufnahme mittels eines vollständigen Strahlungsbündels vorgenommen wird, und dass das hierdurch erhaltene vollständige ähnliche Bild korrigiert wird.

17. Verfahren nach einem der Ansprüche 1 bis 7, dadurch gekennzeichnet, dass die fehlenden Teile eines Grundbildes vervollständigt werden, welches gegebenenfalls durch eine an sich bekannte Interpolation korrigiert wird, die ausgehend von benachbarten Punkten der genannten fehlenden Teile innerhalb des Bildes selbst vorgenommen wird.

# FIG_1

# FIG_2

0 093 649

# FIG_3

13